(19)

Europäisches
Patentamt
European
Patent Office
Office européen
des brevets

(11) **EP 1 769 748 A1**

(12) **EUROPEAN PATENT APPLICATION**
published in accordance with Art. 158(3) EPC

(43) Date of publication:
**04.04.2007 Bulletin 2007/14**

(21) Application number: **05751367.3**

(22) Date of filing: **20.06.2005**

(51) Int Cl.:
***A61B 8/08*** (2006.01)     ***A61B 5/02*** (2006.01)
***A61B 5/107*** (2006.01)

(86) International application number:
**PCT/JP2005/011265**

(87) International publication number:
**WO 2006/001252 (05.01.2006 Gazette 2006/01)**

(84) Designated Contracting States:
**AT BE BG CH CY CZ DE DK EE ES FI FR GB GR
HU IE IS IT LI LT LU MC NL PL PT RO SE SI SK TR**
Designated Extension States:
**AL BA HR LV MK YU**

(30) Priority: **23.06.2004 JP 2004184767**

(71) Applicant: **MATSUSHITA ELECTRIC INDUSTRIAL
CO., LTD.
Osaka 571-8501 (JP)**

(72) Inventors:
• **KATO, Makoto
  Kanagawa 240-0001 (JP)**
• **HAGIWARA, Hisashi
  Kanagawa 227-0066 (JP)**
• **TANNAKA, Yoshinao
  Kanagawa 243-0301 (JP)**

(74) Representative: **Grünecker, Kinkeldey,
Stockmair & Schwanhäusser
Anwaltssozietät
Maximilianstrasse 58
80538 München (DE)**

(54) **BLOOD VESSEL ENDOTHELIUM REACTION MEASURING INSTRUMENT AND METHOD FOR
CONTROLLING BLOOD VESSEL ENDOTHELIUM REACTION MEASURING INSTRUMENT**

(57)     A vascular endothelial reactivity measuring apparatus according to the present invention includes: a blood flow blocking section **2** for blocking blood flow through the arterial blood vessel of an organism; an evaluating section **4** for evaluating the geometric property of the arterial blood vessel or its wall; and a control section **3** for controlling the blood flow blocking section such that the blood flow through the arterial blood vessel is repeatedly blocked and unblocked in at least two iterative blocking/unblocking cycles. During at least a part of a period in which the blood flow through the arterial blood vessel is unblocked, the evaluating section **4** evaluates the geometric property and processes data, representing the geometric property during the partial period, in the iterative blocking/unblocking cycle.

*FIG.2*

EP 1 769 748 A1

**Description**

**TECHNICAL FIELD**

The present invention relates to a vascular endothelial reactivity measuring apparatus for use to make a function test of an arterial blood vessel wall tissue and a method for controlling such a measuring apparatus.

**BACKGROUND ART**

Recently, the number of people suffering from various circulatory system diseases, including heart infarction and brain infarction, has been on the rise, thus making it more and more urgent to prevent and treat these diseases.
The pathopoiesis of heart or brain infarction is closely correlated to arterial sclerosis. More specifically, if an atheroma is created on the arterial wall or if no arterial cells are produced anymore due to various factors such as elevated blood pressure, then the artery loses its elasticity to become hard and fragile. Also, if the blood vessel is clogged up where the atheroma has been created or if a vascular tissue covering the atheroma has ruptured, then the atheroma will move itself into the blood vessel to clog up the artery elsewhere or to rupture the hardened portions of the artery. As a result, these diseases are caused. That is why it is important to diagnose the arterial sclerosis as early as possible to prevent or treat these diseases.
If the arterial sclerosis can be diagnosed early enough to administer some medicine to its patient, then the disease can be treated effectively. However, it is said that once the arterial sclerosis has advanced to a certain degree, the farther advancement of that disease can be checked with the administration of medicine but it is difficult to repair the hardened artery completely.
In the prior art, the lesion of arterial sclerosis is diagnosed by directly observing the inside of the blood vessel with a vascular catheter. However, this diagnosis needs to be carried out with a vascular catheter inserted into the blood vessel of a person under measurement, thus imposing a heavy load on him or her. For that reason, the vascular catheter observation is usually adopted to locate the lesion of arterial sclerosis in a patient who is already known to suffer from that disease but has never been used to make a medical checkup on a supposedly healthy person.
An ultrasonic diagnostic apparatus or an X-ray diagnostic apparatus has been used in the prior art as a noninvasive medical apparatus that imposes only a light load on a person under measurement. Specifically, by irradiating the person with the ultrasonic wave or the x-ray that has been produced externally, geometric information or information about the geometric variation with time of his or her internal body can be acquired without causing pain to him or her. When the information about the geometric variation with time (i.e., mobility information) of an object under measurement in his or her body can be obtained, the attribute information of the object can be obtained. That is to say, the vascular elastic property of the organism can be known and the degree of advancement of the arterial sclerosis can be detected directly.
Among other things, the ultrasonic diagnosis is superior to the X-ray diagnosis because the ultrasonic diagnosis can be made just by putting an ultrasonic probe on a person under measurement. That is to say, in the ultrasonic diagnosis, there is no need to administer a contrast medium to the person under measurement and there is no concern about potential X-ray exposure, either. Also, some ultrasonic diagnostic apparatuses can have significantly improved measuring accuracy thanks to recent remarkable advancement of electronic technologies. As a result, ultrasonic diagnostic apparatuses for measuring the very small motion of a vital tissue have been developed. For example, according to the technique described in Patent Document No. 1, even when vasomotion has an amplitude of just several micrometers, vibration components that may have as high a frequency as several hundreds of Hz can be measured with sufficiently high accuracy. Consequently, the variation in the thickness of the vascular wall or the distortion thereof can be sensed with a high accuracy of several micrometers according to Patent Document No. 1.
Meanwhile, a diagnostic method called a "vascular endothelial function testing" has been researched as a noninvasive method for sensing the degree of advancement of arterial sclerosis. There is a layer of cell groups called "vascular endothelial cells" within an arterial blood vessel. These endothelial cells show various physiological reactions in response to a mechanical stress (i.e., shear stress) caused when blood flows through a blood vessel. Production of nitric oxide (NO) is one of those physiological reactions. Nitric oxide is produced and released by a nitric oxide synthetase to relax (i.e., soften) the plain muscle of the vascular tunica media as an endothelium-derived relaxing factor (EDRF). Also, the function of these vascular endothelial cells is called "endothelium dependent relaxation (EDR)".
It is known that various risk factors including hypertension, hyperlipidemia, smoking and diabetes decrease the EDR. And the decline of this function is said to be among the primary complex of arterial sclerosis. Accordingly, arterial sclerosis should be diagnosed as early as possible by checking the EDR.
Methods for evaluating a variation in the caliber of an arterial blood vessel with an ultrasonic wave before and after blood flow through the arterial blood vessel is blocked are disclosed in Non-patent Document No. 1 and Patent Document No. 2 as EDR checking methods for diagnosing arterial sclerosis. According to the method disclosed in Non-patent Document No. 1, first, blood flow through the artery of an examinee's upper arm is blocked with a cuff at 250 mmHg for five minutes. Thereafter, after the blood flow has been unblocked instantaneously, the caliber of the blood vessel is measured intermittently for several tens of seconds. And arterial sclerosis is diagnosed based on the percentage of increase in the caliber of the blood vessel. On the other hand, according to the method disclosed in Patent Document No. 2, the caliber of the blood vessel of a resting examinee is measured and then blood flow through the artery of his or her lower arm is blocked for five minutes. After the blood flow has been unblocked, the maximum vascular caliber is measured intermittently for about two minutes and a flow mediated dilation (FMD) value is obtained based on the vascular caliber and used as an index to arterial sclerosis.

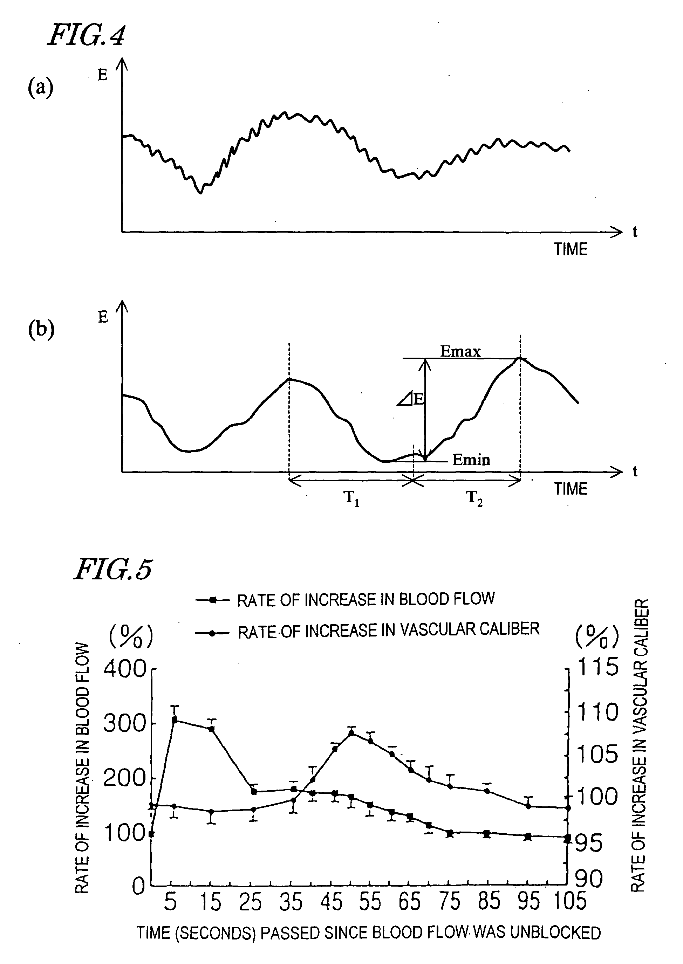

According to the method disclosed in Non-patent Document No. 1, the vascular caliber is measured by reading the distances between m-lines (i.e., the distance from the intermediate point between the tunica media and the adventitious tunica of the front wall to the intermediate point between the tunica media and the adventitious tunica of the rear wall) on a 0.1 mm basis in an image obtained by an ultrasonic diagnostic apparatus to show a major-axis cross section of the blood vessel. And the average of four to six measured values is calculated as the vascular caliber. FIG. 5 shows the results of tests that were carried out on nine male examinees. In FIG. 5, the solid squares plot the rate of increase in the amount of blood flowing through the right upper arm artery after the blood flow in the right forearm was unblocked, while the solid circles plot the rate of increase in the caliber of the right upper arm artery compared to that of a resting examinee. Also, in FIG. 5, the abscissa represents the time passed since the blood flow was unblocked, the ordinate on the left-hand side represents the rate of increase in the amount of blood flowing, and the ordinate on the right-hand side represents the rate of increase in the vascular caliber. As shown in FIG. 5, after the blood flow has been unblocked, the amount of blood flowing increases temporarily but gradually decreases with time after that. When the amount of blood flowing increases temporarily after the blood flow has been unblocked, nitric oxide is produced in response to that stimulus and relaxes the plain muscle of the tunica media of the vascular wall. As a result, the blood vessel stretches. As can be seen from FIG. 5, the vascular caliber increases significantly in about 45 to 60 seconds after the blood flow was unblocked as compared to that of a resting examinee. The rate of increase in vascular caliber is about 6%. According to Non-patent Document No. 1 if the rate of increase in vascular caliber is below 3 or 4%, then the chances of arterial sclerosis are rather high.

Patent Document No. 1: Japanese Patent Application Laid-Open Publication No. 10-5226
Patent Document No. 2: Japanese Patent Application Laid-Open Publication No. 2003-245280
Non-patent Document No. 1: Masayoshi Hashimoto, Yasuyoshi Ohuchi, "Vascular Compliance Test", Magazine of the Japan Medical Association, Vol. 120, No. 8, Oct. 15 1998, pp. S93-S96

## DISCLOSURE OF INVENTION

## PROBLEM TO BE SOLVED BY THE INVENTION

According to the methods disclosed in Non-patent Document No. 1 and Patent Document No. 2 mentioned above, a blood flow blocking period of five minutes and a measuring period of a few more minutes are needed. Thus, it takes about seven minutes in total to make a single measurement. Besides, some more time for preparing for the measurement also needs to be taken into account. On top of that, the vascular caliber is measured on a 0.1 mm basis according to the method disclosed in Non-patent Document No. 1. However, since the vascular caliber of the upper arm artery is about 3 mm, the error is as high as about 3%. That is to say, the measuring accuracy realized by that method is far from satisfactory.

In order to overcome the problems described above, an object of the present invention is to provide a vascular endothelial reactivity measuring apparatus that can carry out high-reliability measurements in a sufficiently short time.

## MEANS TO SOLBE THE PROBLEM

A vascular endothelial reactivity measuring apparatus according to the present invention includes: a blood flow blocking section for blocking blood flow through the arterial blood vessel of an organism; an evaluating section for evaluating the geometric property of the arterial blood vessel or its wall; and a control section for controlling the blood flow blocking section such that the blood flow through the arterial blood vessel is repeatedly blocked and unblocked in at least two iterative blocking/unblocking cycles. During at least a part of a period in which the blood flow through the arterial blood vessel is unblocked, the evaluating section evaluates the geometric property and processes data, representing the geometric property during the partial period, by utilizing the iterative blocking/unblocking cycle.

In one preferred embodiment, the evaluating section further evaluates the attribute property of the blood vessel wall based on the geometric property evaluated.

In another preferred embodiment, the evaluating section extracts components that change synchronously with the iterative blocking/unblocking cycle from the data representing the geometric property and/or attribute property during the partial period.

In another preferred embodiment, the evaluating section superposes multiple sets of data, each representing the geometric property and/or the attribute property to be assessed in every iterative blocking/unblocking cycle, and evaluates the geometric property and/or the attribute property based on the superposed data.

In another preferred embodiment, the evaluating section subjects the data representing the geometric property and/or the attribute property to Fourier transform, extracts only iterative frequency components of the blocking/unblocking cycle, and evaluates the geometric property and/or the attribute property based on the data extracted.

In another preferred embodiment, the evaluating section includes a bandpass filter that has a property to pass frequency components, of which the period is an integral multiple of the iterative blocking/unblocking cycle, to extract the data.

In a specific preferred embodiment, the evaluating section is an ultrasonic diagnostic apparatus.

In another specific preferred embodiment, the evaluating section is an X-ray diagnostic apparatus.

In yet another preferred embodiment, the evaluating section is a nuclear magnetic resonance diagnostic apparatus.

In yet another preferred embodiment, the geometric property evaluated by the evaluating section is the thickness of the blood vessel wall and/or a variation in the thickness of the blood vessel wall.

In yet another preferred embodiment, the geometric property evaluated by the evaluating section is the caliber of the blood vessel and/or a variation in the caliber of the blood vessel.

In yet another preferred embodiment, the attribute property evaluated by the evaluating section is the elastic property of the blood vessel wall.

A control method according to the present invention is a method for getting a vascular endothelial reactivity measuring apparatus controlled by a control section of the vascular endothelial reactivity measuring apparatus. The method includes the steps of: evaluating the geometric property of an arterial blood vessel or its wall during at least a part of a period in which blood flow through the arterial blood vessel is unblocked while the blood flow through the arterial blood vessel is repeatedly blocked and unblocked in at least two iterative blocking/unblocking cycles; and processing data, representing the geometric property during the partial period, by utilizing the iterative blocking/unblocking cycle.

In one preferred embodiment, the step of processing includes extracting components that change synchronously with the iterative blocking/unblocking cycle from the data representing the geometric property.

In another preferred embodiment, the method further includes the step of evaluating the attribute property of the blood vessel or its wall based on the geometric property evaluated during the partial period, and the step of processing includes processing the data representing the geometric property and the data representing the attribute property in the iterative blocking/unblocking cycle.

In another preferred embodiment, the step of processing includes extracting components that change synchronously with the iterative blocking/unblocking cycle from the data representing the geometric property and the data representing the attribute property.

In another preferred embodiment, the step of processing includes superposing multiple sets of data, each representing the geometric property and/or the attribute property to be assessed in every iterative blocking/unblocking cycle, and evaluating the geometric property and/or the attribute property based on the superposed data.

In another preferred embodiment, the step of processing includes subjecting the data representing the geometric property and/or the attribute property to Fourier transform, extracting only iterative frequency components of the blocking/unblocking cycle, and evaluating the geometric property and/or the attribute property based on the data extracted.

In another preferred embodiment, the step of processing includes extracting the data by using a bandpass filter that has a property to pass frequency components, of which the period is an integral multiple of the iterative blocking/unblocking cycle.

In another preferred embodiment, the step of evaluating includes evaluating the geometric property with an ultrasonic diagnostic apparatus, an X-ray diagnostic apparatus or a nuclear magnetic resonance diagnostic apparatus.

## EFFECT OF THE INVENTION

A vascular endothelial reactivity measuring apparatus according to the present invention repeatedly blocks and unblocks the blood flow in at least two iterative cycles and processes the evaluated data by utilizing the blocking/unblocking cycle. Consequently, the resultant geometric property is accurate enough and hardly affected by noise. In addition, by taking advantage of the iterative cycle, the blood flow blocking period can be shortened. As a result, it takes a shorter time to make a diagnosis using a vascular endothelial reactivity measuring apparatus.

## BRIEF DESCRIPTION OF THE DRAWINGS

FIG. **1** is a block diagram schematically showing a configuration for a vascular endothelial reactivity measuring apparatus according to the present invention.

FIG. **2** illustrates how to evaluate the vascular endothelial reactivity with the vascular endothelial reactivity measuring apparatus of the present invention.

FIG. **3** is a block diagram showing a configuration for an ultrasonic diagnostic apparatus for use in the vascular endothelial reactivity measuring apparatus shown in FIG. **2**.

FIGS. **4(a)** and **4(b)** are graphs showing the elastic moduli that were measured with the vascular endothelial reactivity measuring apparatus shown in FIG. **2** in a situation where components changing synchronously with the iterative blocking/unblocking cycle were not extracted and in a situation where those components were extracted, respectively.

FIG. **5** is a graph showing a variation in the amount of blood flowing and a variation in vascular caliber after the blood flow has been unblocked to evaluate the vascular endothelial reactivity.

## DESCRIPTION OF REFERENCE NUMERALS

| | |
|---|---|
| **1** | vascular endothelial reactivity measuring apparatus |
| **2** | blocking section |
| **3** | control section |
| **4** | evaluating section |
| **10** | cuff |
| **11** | arm |
| **12** | cuff control section |
| **13** | ultrasonic diagnostic apparatus |
| **14** | ultrasonic probe |
| **16** | monitor |
| **17** | arterial blood vessel |
| **20** | computed data storage section |
| **21** | DSC |
| **22** | display control section |
| **23** | ultrasonic wave transmitting/receiving section |
| **24** | CPU |
| **25** | time delay control section |
| **26** | delay data storage section |
| **27** | phase detecting section |
| **28** | filter |
| **29** | computing section |
| **34** | blood pressure manomeasuring apparatus |

## BEST MODE FOR CARRYING OUT THE INVENTION

Hereinafter, preferred embodiments of a vascular endothelial reactivity measuring apparatus according to the present invention will be described.

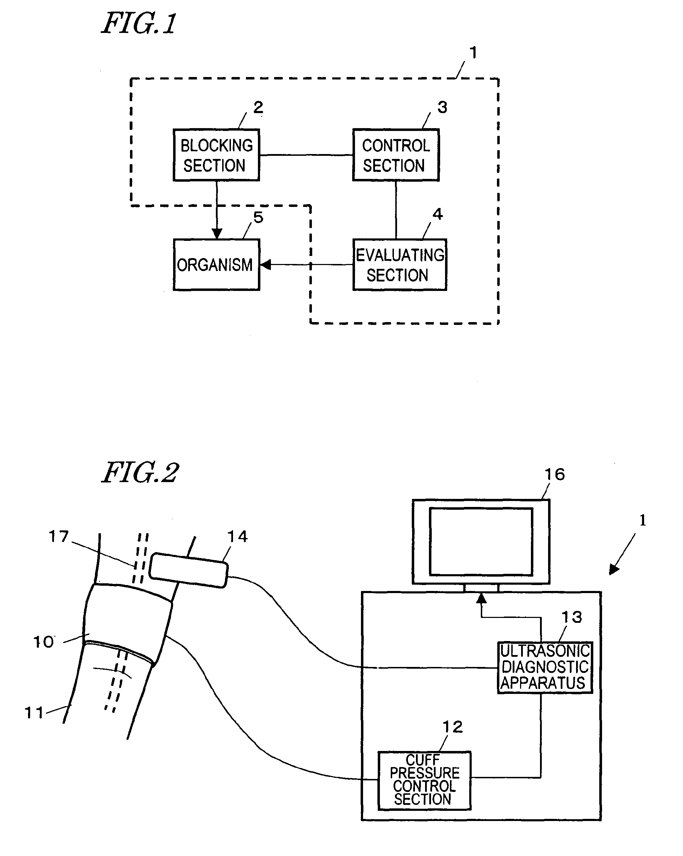

FIG. **1** is a block diagram schematically showing a configuration for a vascular endothelial reactivity measuring apparatus 1. The measuring apparatus **1** includes a blood flow blocking section **2,** a control section **3** and an evaluating section **4**. The blocking section **2** may be a cuff (i.e., a tourniquet) that uses air pressure and is put on an arm of a person under measurement. Specifically, under the control of the control section **3,** air is pumped into the cuff, thereby compressing the artery in the arm and blocking the blood flow through the artery. Conversely, also under the control of the control section **3,** the air may be pumped out of the cuff, thereby unblocking the blood flow through the artery. This vascular endothelial reactivity measuring apparatus evaluates the geometric property of the vascular wall after the blood flow has been unblocked. That is why the blood flow is preferably unblocked quickly under the control of the control section **3**. Optionally, a known blood pressure manometer may be used as the blocking section **2**.

The control section **3** instructs the blocking section **2** to either block or unblock the blood flow at predetermined timings, which will be described in detail later.

The evaluating section **4** evaluates the geometric property of the arterial blood vessel, in which the blood flow is blocked by the blocking section **2**, or its wall. The geometric property evaluated includes the caliber of the blood vessel, the thickness of the vascular wall and a variation in the thickness of that vascular wall. Optionally, the elasticity modulus, distortion, viscosity and other attribute properties of the vascular wall may further be obtained based on the geometric property. The evaluation is preferably carried out using the ultrasonic wave, the X-ray or the nuclear magnetic resonance. Therefore, an ultrasonic diagnostic apparatus, an X-ray diagnostic apparatus or a nuclear magnetic resonance diagnostic apparatus is preferably used as the evaluating section **4**. Among other things, an ultrasonic diagnostic apparatus is particularly preferred as the evaluating section **4** because ultrasonic waves hardly affect human bodies.

Next, the blocking/unblocking timings will be described. The vascular endothelial reactivity measuring apparatus **1** evaluates the vascular endothelial reactivity (or the EDR among other things). For that purpose, a state in which the production of nitric oxide is checked and a state in which the production of nitric oxide is promoted need to be defined for the endothelial cells. That is to say, the amount of nitric oxide produced is changed by blocking and unblocking the blood flow and the degree of relaxation of the plain muscle caused by nitric oxide is estimated by evaluating the geometric property.

The amount of time it takes to sense a significant difference in the geometric property of the blood vessel before and after the blood flow is unblocked depends on the manifestation time of the EDR. As can be seen from FIG. 5, the vascular caliber maximizes in 45 to 60 seconds after the blood flow has been unblocked. Accordingly, the unblocking period for obtaining the maximum vascular caliber is preferably about 60 seconds. However, the present invention can be carried out not just in that particular situation where the vascular caliber has maximized but also in a situation where the vascular caliber has just increased. For that reason, there is no need to put measuring on hold for as many as 45 seconds after the blood flow has been unblocked. The present inventors discovered that the vascular caliber increased significantly in about 30 seconds after the blood flow was unblocked. That is why the unblocking period is preferably 30 seconds to 60 seconds. During at least a part of this unblocking period, the geometric property of the blood vessel or its wall is evaluated.

Meanwhile, checking the production of nitric oxide is affected by various conditions resulting in individual differences such as the magnitude of pressure caused by the blocking section **2** on the artery, the location of the artery and the blood pressure. That is why it is difficult to set a generally preferred length of the blocking period. However, it is at least true that the longer the blocking period, the more perfectly the production of nitric oxide can be checked. In view of this consideration, the blocking period is set to several tens of seconds to several minutes in this preferred embodiment. More particularly, the blocking period is preferably 30 seconds to 3 minutes.

The blocking period and unblocking period are defined as described above and the blocking and unblocking operations are repeatedly carried out in at least two cycles (preferably in four or more cycles). And during at least a part of the unblocking period in every cycle, the geometric property of the blood vessel or its wall is evaluated. Optionally, the geometric property of the blood vessel or its wall may be evaluated during the blocking period, too. Then, the geometric property data thus obtained or data representing the attribute property obtained from the geometric property is processed by utilizing the iterative blocking/unblocking cycle.

For example, multiple sets of geometric property (or attribute property) data, each of which is acquired in every blocking/unblocking cycle, are superposed one upon the other and the geometric property during the unblocking period may be evaluated based on the superposed data. By performing such computations, only components that are dependent on the iterative blocking/unblocking cycle are enhanced and the other components, which are not dependent on the iterative cycle, cancel each other. Alternatively, only components that change synchronously with the iterative blocking/unblocking cycle are extracted from the geometric property data acquired during the two or more unblocking periods, and the geometric property value of the blood vessel or its wall during the blocking period is derived from the data extracted. For that purpose, the geometric property data may be subjected to Fourier transform to extract only the iterative frequency components of the blocking/unblocking cycle. As another alternative, a bandpass filter, having a property to pass frequencies of which the period is n times as long as (where n is an integer that is equal to or greater than one) the blocking/unblocking cycle, may be obtained through computations and data may be extracted by passing data through the bandpass filter.

According to this method, the data is processed in the blocking/unblocking cycle. Thus, various external factors that might decrease the reproducibility of measuring (including noise caused when the blocking section 2 or evaluating section **4** shifts from the measuring spot of the person under measurement and noise caused by his or her respiration) can be eliminated. As a result, the geometric property can be evaluated more accurately. A conventional method for evaluating vascular endothelial reactivity is susceptible to these types of noise so easily as to commit significant measuring errors. Also, to check the production of nitric oxide as perfectly as possible and to magnify the variation in geometric property due to the production of nitric oxide as much as possible, a relatively long blocking period of about five minutes is usually adopted. In contrast, the vascular endothelial reactivity measuring apparatus of the present invention can reduce the influence of noise to such a level as to produce just minor measuring errors. In addition, even if the variation in geometric property is a barely sensible one, the measuring apparatus can still produce significant measuring results. As a result, the blocking period can be shortened. A long blocking period may be a taboo for some examinees. That is why a shortened blocking period has a great significance.

The geometric property or attribute property evaluated in this manner shows a variation corresponding to the amount of nitric oxide produced. As described above, the EDR, which is a reaction to the production of nitric oxide, decreases due to a risk factor such as hypertension, hyperlipidemia, smoking or diabetes. Accordingly, evaluation of the geometric property or attribute property may be begun with a property that was diagnosed as arterial sclerosis caused by any of these risk factors.

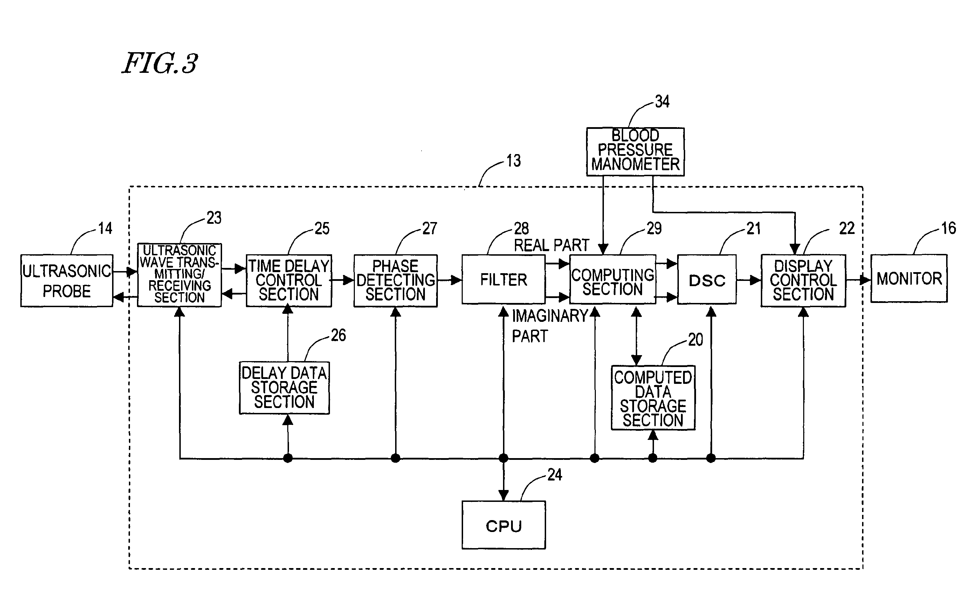

Hereinafter, a specific example of the vascular endothelial reactivity measuring apparatus will be described. The vascular endothelial reactivity measuring apparatus to be described below evaluates the geometric property of a blood vessel wall using an ultrasonic wave. FIG. 2 schematically illustrates how to evaluate the geometric property of the arterial blood vessel 17 in an arm 11 of an examinee by using the vascular endothelial reactivity measuring apparatus 1. The vascular endothelial reactivity measuring apparatus 1 includes a cuff 10, a cuff pressure control section 12 (which is a control section for controlling the cuff 10), and an ultrasonic diagnostic apparatus 13. The cuff 10 and the cuff pressure control section 12 together functions as the blood flow blocking section 2, while the ultrasonic diagnostic apparatus 13 functions as the evaluating section 4. Also, the CPU 24 built in the ultrasonic diagnostic apparatus 13 (see FIG. 3) functions as the control section 3. A blood pressure manometer or a portion thereof may be used as the cuff 10. An ultrasonic probe 14 is connected to the ultrasonic diagnostic apparatus 13.

First, to evaluate the geometric property of the arterial blood vessel 17, the cuff 10 is wrapped around the upper portion of the arm 11. Also, the ultrasonic probe 14 is arranged closer to the heart than the cuff 10 is so as to evaluate the geometric property of the arterial blood vessel 17, too. In the example illustrated in FIG. 2, the ultrasonic probe 14 is arranged closer to the heart than the cuff 10 is. Alternatively, the cuff 10 may be arranged closer to the heart than the ultrasonic probe 14 is.

The cuff pressure control section 12 controls the cuff 10 such that the blood flow is repeatedly and cyclically blocked and unblocked at predetermined intervals. For example, air may be pumped into and out of the cuff 10 such that the blood flow is blocked for 60 seconds and then unblocked for another 60 seconds. The blocking pressure may be 200 mmHg, for example.

The ultrasonic diagnostic apparatus 13 receives an unblocking signal from the cuff pressure control section 12 and evaluates the geometric property of the arterial blood vessel 17 by using the ultrasonic probe 14 during at least a part of the unblocking period. An echo signal obtained by the ultrasonic probe 14 from the arterial blood vessel 17 is processed by the ultrasonic diagnostic apparatus 13, thereby sensing the variation in the caliber of the arterial blood vessel 17 or the variation in the thickness of the blood vessel wall quantitatively. Optionally, a B-mode diagnostic image may also be generated based on the echo signal. For that purpose, a monitor 16 may be connected to the ultrasonic diagnostic apparatus 13 so as to present a B-mode tomographic image thereon. The vascular caliber, the thickness of the blood vessel wall or their variations may be either directly read from the B-mode image or figured out by a zero-cross method by which the displacement of an object is calculated based on the zero-cross point shift time of an RF signal. A phase difference tracking by a restricted minimum square method as disclosed in Patent Document No. 1 is a preferred measuring method for the ultrasonic diagnostic apparatus 13 of the present invention because the vascular caliber and the variation in the thickness of the blood vessel can be measured highly accurately.

FIG. 3 is a block diagram showing a configuration for an ultrasonic diagnostic apparatus 13 that uses a high-precision tracking method. The ultrasonic diagnostic apparatus 13 includes an ultrasonic wave transmitting/receiving section 23, a CPU 24, a time delay control section 25, a delay data storage section 26, a phase detecting section 27, a filter 28, a computing section 29, a computed data storage section 20, a DSC 21 and a display control section 22.

The ultrasonic wave transmitting/receiving section 23 includes a driver for driving the ultrasonic probe 14 as an ultrasonic probe driving section and a receiver for amplifying the ultrasonic reflected wave as a receiving section. Under the control of the CPU 24, which controls the overall ultrasonic diagnostic apparatus 13, the ultrasonic probe driver applies a predetermined drive pulse signal to the ultrasonic probe 14. An ultrasonic transmitted wave, transmitted by the ultrasonic probe 14 in response to the drive pulse signal, is reflected by an organism to produce an ultrasonic reflected wave, which is then received at the ultrasonic probe 14. The ultrasonic reflected wave is received by the ultrasonic probe 14 and then amplified by the receiver. The ultrasonic transmitting/receiving section 23 further includes an A/D converter for converting the ultrasonic reflected wave, amplified by the receiver, into a digital signal.

The time delay control section 25 is connected to the ultrasonic wave transmitting/receiving section 23 in order to control the time delay of the drive pulse signal to be supplied from the ultrasonic wave transmitting/receiving section 23 to a group of ultrasonic vibrators in the ultrasonic probe 14. In this manner, the ultrasonic transmitted wave to be transmitted from the ultrasonic probe 14 can have its acoustic line direction and depth of focus changed. Also, by controlling the time delay of an ultrasonic reflected wave signal that has been received by the ultrasonic probe 14 and then amplified by the ultrasonic wave transmitting/receiving section 23, the acoustic line direction of the ultrasonic wave to receive can be changed.

The phase detecting section 27 detects the phase of the received reflected wave signal, of which the time delay has been controlled by the time delay control section 25, thereby splitting the signal into a real part signal and an imaginary part signal, which are then input to the filter 28. The filter 28 filters out components that have not been reflected by the vascular wall.

The output of the filter 28 is supplied to the computing section 29. The computing section 29 includes a motion velocity calculating section, a position calculating section, a shrinkage/stretch calculating section, and an elasticity modulus calculating section. The motion velocity calculating section calculates the motion velocity of the blood vessel as the object by using the real part and imaginary part signals of the phase-detected signal. More specifically, the motion velocity calculating section sets a plurality of measuring points on the acoustic line of an ultrasonic wave to be transmitted from the ultrasonic probe **14.** Under the restriction that the amplitude does not change, but only the phase and reflection spot change, between the reflected wave signal r(t) at a time t and another reflected wave signal r(t + Δt) obtained after a very small amount of time Δt, the computing section **29** calculates the phase difference by a minimum square method so as to minimize the waveform mismatch between the reflected wave signals r(t) and r(t+Δt). The motion velocity of each measuring point is derived from this phase difference and then integrated by the position calculating section and the shrinkage/stretch calculating section, thereby obtaining the magnitudes of positional displacement and shrinkage/ stretch. By choosing two spots corresponding to the thickness of a vascular wall from the multiple measuring points set, the thickness or a variation in the thickness of the vascular wall can be obtained as the geometric property of the vascular wall.

Also, the computing section **29** can effectively evaluate the attribute property of the vascular wall tissue based on the geometric property evaluated. The elastic property **Er** of the vascular wall in the radial direction is given by

$$\mathbf{Er} = \Delta \mathbf{p} / (\Delta \mathbf{h} / \mathbf{h})$$

where Δh is the maximum variation in the thickness of the vascular wall within one heart beat, Δp is the difference between the maximum and minimum blood pressure values, and h is the thickness of the vascular wall. To measure the maximum and minimum blood pressure values, the blood pressure of the examinee may be measured with the blood pressure manometer **34** and the measured values may be input to the computing section **29.** The cuff of the blood pressure manometer **34** may also function as the cuff **10** for blocking the blood flow. Furthermore, by using the radius r of the blood vessel, the elastic property **Eθ** in the circumferential direction can be given by

$$\mathbf{E}\theta = 1/2[(\mathbf{r}/\mathbf{h}) + 1] \times \Delta \mathbf{p} / (\Delta \mathbf{h} / \mathbf{h}).$$

Also, by using the variation in vascular caliber, the rate of increase in vascular caliber compared to the value of a resting examinee, i.e., an FMD value, can be obtained as follows:

$$\mathbf{FMD} = \Delta \mathbf{d} \times 100 / \mathbf{d}$$

where d is the vascular caliber of a resting examinee and Δd is the maximum variation in vascular caliber.

When the computing section **29** figures out the geometric property or attribute property of the arterial blood vessel **17** or its wall, only those components changing synchronously with the iterative blocking/unblocking cycle are preferably extracted by either Fourier transform or bandpass filtering processing. Then, the geometric property of the arterial blood vessel **17** or the attribute property of the vascular wall is preferably evaluated based on the data extracted. As a result, the measuring can be carried out even more accurately.

Optionally, to make a two-dimensional mapping display, the geometric property or attribute property thus obtained may be converted by the DSC **21** into an image format suited for presenting it on the display **16** and then presented on the display **16.**

FIGS. **4(a)** and **4(b)** are graphs showing a variation in the elastic property **E** of the vascular wall in the radial direction with time as evaluated by the vascular endothelial reactivity measuring apparatus **1** including the ultrasonic diagnostic apparatus **13** shown in FIG. **3.** In the graph shown in FIG. **4(b),** the computing section **29** figured out the elastic property by extracting only components changing synchronously with the iterative blocking/unblocking cycle. In the graph shown in FIG. **4(a)** on the other hand, the elastic property was figured out without extracting those components changing synchronously with the iterative blocking/unblocking cycle for the purpose of comparison. As can be seen from FIG. **4(a),** the resultant graph representing the elastic property is a superposition of a lot of small waveforms. Such a graph was obtained due to the displacement of the probe **14** that was once released by, and then got hold of again, by the operator, the examinee's respiration and other subtle motions, or electromagnetic noise.

In FIG. **4(b),** T2 represents a period in which blood flow through the blood vessel was blocked, while T1 represents a period in which the blood flow was unblocked. When the blood flow is blocked, the amount of nitric oxide produced decreases. As a result, the vascular wall hardens and the elastic property has an increased value while the blood flow is being blocked. Meanwhile, once the blood flow has been unblocked, nitric oxide is produced profusely, thus softening the vascular wall and decreasing the elastic property value.

To extract only those components changing synchronously with the iterative blocking/unblocking cycle, a bandpass filter, which can sufficiently pass a frequency **f1,** of which one period is twice as long as one unblocking period **T1** (i.e., f1=1/(2·T1)), and a frequency **f2,** of which one period is twice as long as one blocking period **T2** (i.e., f2=1/(2·T2)), is used such that the computing section **29** can remove noise and other external variation components.

As can be seen from FIGS. **4(a)** and **4(b),** by extracting only those components changing synchronously with the iterative blocking/unblocking cycle, the noise and other external variation components are removed. Thus, it can be seen from the graph of FIG. **4(b)** that the maximum and minimum values **Emax** and **Emin** of the elastic property **E** and their difference Δ**E** can be easily determined and the elastic property can be evaluated accurately. Not just ΔE but also the ratio of the maximum value **Emax** to the minimum value **Emin** or the rate of increase of ΔE (i.e., (Emax-Emin)/Emax) may be used effectively as an index for evaluating the vascular endothelial reactivity.

In the preferred embodiment described above, the ultrasonic diagnostic apparatus shown in FIG. **3** is used as an exemplary evaluating section. However, the evaluating section may have any other configuration as long as the evaluating section can evaluate the geometric property and attribute property of the arterial blood vessel or its wall. Also, in the preferred embodiment described above, the elastic property is evaluated as one of attribute properties of the vascular wall tissue. Alternatively, the vascular endothelial reactivity can also be evaluated similarly by compliance, which is the reverse number of the elastic property.

## INDUSTRIAL APPLICABILITY

A vascular endothelial reactivity measuring apparatus according to the present invention realizes high-reliability measurements in just a short time and can be used effectively in the medical and health care fields.

## Claims

1.   A vascular endothelial reactivity measuring apparatus comprising:

   a blood flow blocking section for blocking blood flow through the arterial blood vessel of an organism;
   an evaluating section for evaluating the geometric property of the arterial blood vessel or its wall; and
   a control section for controlling the blood flow blocking section such that the blood flow through the arterial blood vessel is repeatedly blocked and unblocked in at least two iterative blocking/unblocking cycles,

   wherein during at least a part of a period in which the blood flow through the arterial blood vessel is unblocked, the evaluating section evaluates the geometric property and processes data, representing the geometric property during the partial period, by utilizing the iterative blocking/unblocking cycle.

2.   The vascular endothelial reactivity measuring apparatus of claim 1, wherein the evaluating section further evaluates the attribute property of the blood vessel wall based on the geometric property evaluated.

3.   The vascular endothelial reactivity measuring apparatus of claim 1 or 2, wherein the evaluating section extracts components that change synchronously with the iterative blocking/unblocking cycle from the data representing the geometric property and/or attribute property during the partial period.

4.   The vascular endothelial reactivity measuring apparatus of claim 1 or 2, wherein the evaluating section superposes multiple sets of data, each representing the geometric property and/or the attribute property to be assessed in every iterative blocking/unblocking cycle, and evaluates the geometric property and/or the attribute property based on the superposed data.

5.   The vascular endothelial reactivity measuring apparatus of claim 3, wherein the evaluating section subjects the data representing the geometric property and/or the attribute property to Fourier transform, extracts only iterative frequency components of the blocking/unblocking cycle, and evaluates the geometric property and/or the attribute property based on the data extracted.

6.   The vascular endothelial reactivity measuring apparatus of claim 3, wherein the evaluating section includes a bandpass filter that has a property to pass frequency components, of which the period is an integral multiple of the iterative blocking/unblocking cycle, to extract the data.

7. The vascular endothelial reactivity measuring apparatus of one of claims 1 to 6, wherein the evaluating section is an ultrasonic diagnostic apparatus.

8. The vascular endothelial reactivity measuring apparatus of one of claims 1 to 6, wherein the evaluating section is an X-ray diagnostic apparatus.

9. The vascular endothelial reactivity measuring apparatus of one of claims 1 to 6, wherein the evaluating section is a nuclear magnetic resonance diagnostic apparatus.

10. The vascular endothelial reactivity measuring apparatus of one of claims 1 to 9, wherein the geometric property evaluated by the evaluating section is the thickness of the blood vessel wall and/or a variation in the thickness of the blood vessel wall.

11. The vascular endothelial reactivity measuring apparatus of one of claims 1 to 9, wherein the geometric property evaluated by the evaluating section is the caliber of the blood vessel and/or a variation in the caliber of the blood vessel.

12. The vascular endothelial reactivity measuring apparatus of one of claims 2 to 9, wherein the attribute property evaluated by the evaluating section is the elastic property of the blood vessel wall.

13. A method for getting a vascular endothelial reactivity measuring apparatus controlled by a control section of the measuring apparatus, the method comprising the steps of:

evaluating the geometric property of an arterial blood vessel or its wall during at least a part of a period in which blood flow through the arterial blood vessel is unblocked while the blood flow through the arterial blood vessel is repeatedly blocked and unblocked in at least two iterative blocking/unblocking cycles; and
processing data, representing the geometric property during the partial period, by utilizing the iterative blocking/unblocking cycle.

14. The method of claim 13, wherein the step of processing includes extracting components that change synchronously with the iterative blocking/unblocking cycle from the data representing the geometric property.

15. The method of claim 13, further comprising the step of evaluating the attribute property of the blood vessel or its wall based on the geometric property evaluated during the partial period, and
wherein the step of processing includes processing the data representing the geometric property and the data representing the attribute property in the iterative blocking/unblocking cycle.

16. The method of claim 15, wherein the step of processing includes extracting components that change synchronously with the iterative blocking/unblocking cycle from the data representing the geometric property and the data representing the attribute property.

17. The method of one of claims 13 to 15, wherein the step of processing includes superposing multiple sets of data, each representing the geometric property and/or the attribute property to be assessed in every iterative blocking/unblocking cycle, and evaluating the geometric property and/or the attribute property based on the superposed data.

18. The method of one of claim 14 to 16, wherein the step of processing includes subjecting the data representing the geometric property and/or the attribute property to Fourier transform, extracting only iterative frequency components of the blocking/unblocking cycle, and evaluating the geometric property and/or the attribute property based on the data extracted.

19. The method of one of claim 14 to 16, wherein the step of processing includes extracting the data by using a bandpass filter that has a property to pass frequency components, of which the period is an integral multiple of the iterative blocking/unblocking cycle.

20. The method of claim 13, wherein the step of evaluating includes evaluating the geometric property with an ultrasonic diagnostic apparatus, an X-ray diagnostic apparatus or a nuclear magnetic resonance diagnostic apparatus.

## FIG.1

## FIG.2

FIG.3

*FIG.4*

(a)

(b)

*FIG.5*

TIME (SECONDS) PASSED SINCE BLOOD FLOW WAS UNBLOCKED

## INTERNATIONAL SEARCH REPORT

| | International application No. |
|---|---|
| | PCT/JP2005/011265 |

**A. CLASSIFICATION OF SUBJECT MATTER**
Int.Cl⁷ A61B8/08, 5/02, 5/107

According to International Patent Classification (IPC) or to both national classification and IPC

**B. FIELDS SEARCHED**

Minimum documentation searched (classification system followed by classification symbols)
Int.Cl⁷ A61B8/00-8/12, 5/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | Masayoshi HASHIMOTO et al., "Kekkan Shintensei Kensa", The Journal of the Japan Medical Association, 15 October, 1998 (15.10.98), Vol.120, No.8, pages S93 to S96 | 1-20 |
| A | JP 2003-180690 A (Mediakurosu Kabushiki Kaisha, Kabushiki Kaisha Sofuto Medikaru), 02 July, 2003 (02.07.03), Full text; all drawings (Family: none) | 1-20 |
| A | JP 2002-224063 A (Aloka Co., Ltd.), 13 August, 2002 (13.08.02), Full text; all drawings (Family: none) | 1-20 |

☒ Further documents are listed in the continuation of Box C. ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 17 August, 2005 (17.08.05) | 20 September, 2005 (20.09.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No.

PCT/JP2005/011265

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2003-144395 A  (Fukuda Denshi Co., Ltd.),<br>20 May, 2003 (20.05.03),<br>Full text; all drawings<br>(Family: none) | 1-20 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10005226 A

- JP 2003245280 A

**Non-patent literature cited in the description**

- **MASAYOSHI HASHIMOTO ; YASUYOSHI OHUCHI.** Vascular Compliance Test. *Magazine of the Japan Medical Association,* 15 October 1998, vol. 120 (8), S93-S96